# EUROPEAN PATENT APPLICATION

(11) **EP 4 246 494 A1**
(43) Date of publication of application: **20.09.2023**
(21) Application number: 23158722.1
(22) Date of filing: 27.02.2023
(51) Int. Cl.: G08G 5/00, A61B 5/18, B60W 40/08

(54) **MULTIMODAL PILOT MONITORING METHODS AND SYSTEMS**

(30) Priority: 17.03.2022 IN 202211014831; 29.04.2022 US 202217661380
(71) Applicant: Honeywell International Inc., Charlotte, NC 28202 (US)
(72) Inventor: FEYEREISEN, Thea, Charlotte, 28202 (US); HE, Gang, Charlotte, 28202 (US); SAPTHARISHI, Sivaraman, Charlotte, 28202 (US); ATASSI, Hicham, Charlotte, 28202 (US); MOHAMMED, Nasiruddin, Charlotte, 28202 (US); KOSIK, Michal, Charlotte, 28202 (US)
(74) Representative: LKGlobal UK Ltd.

(57) **Abstract**

Methods and systems are provided for assisting operation of a vehicle by monitoring a physiological state of an operator and automatically initiating a remedial action responsive to a change in the physiological state. One method involves determining a biometric indication of the current physiological state based on measurement data from one or more sensing devices, determining an activity-based indication of the current physiological state based on output signals from one or more user interface elements and determining an aggregate indicator of the current physiological state as a function of the biometric indication and the activity-based indication using one or more weighting factors that vary during operation of the vehicle. One or more remedial actions are automatically initiated based on the aggregate indicator, for example, in response to a change in physiological state indicated by the value of the aggregate indicator.

## Description

### CROSS-REFERENCE TO RELATED APPLICATION

This application claims priority to India Provisional Patent Application No. 202211014831, filed March 17, 2022, the entire content of which is incorporated by reference herein.

### TECHNICAL FIELD

The subject matter described herein relates generally to vehicle systems, and more particularly, embodiments of the subject matter relate to multimodal methods and systems for monitoring the physiological state of a pilot or other vehicle operator.

### BACKGROUND

Various forms of automation have been incorporated into vehicles to improve operations and reduce stress, fatigue, and other potential contributing factors for human error. For example, many modern aircraft incorporate a flight management system (FMS) and other avionics systems capable of providing autopilot functionality and other automated vehicle operations. While various forms of automation have been incorporated into vehicles such as aircraft, a vehicle operator often has to manually operate the vehicle in response to abnormal events or various other conditions or scenarios. However, in some situations, a pilot or other vehicle operator may become distracted, incapacitated or otherwise impaired with respect to his or her ability to operate the vehicle (e.g., due to workload, sleep or drowsiness, loss of situational awareness, health emergencies, etc.). Accordingly, it is desirable to provide aircraft systems and methods for mitigating potential pilot incapacity or other inability to fully operate the aircraft. Other desirable features and characteristics of the methods and systems will become apparent from the subsequent detailed description and the appended claims, taken in conjunction with the accompanying drawings and the preceding background.

### BRIEF SUMMARY

Methods and systems are provided for assisting operation of a vehicle. One method involves determining a first value corresponding to a first physiological state of a plurality of different physiological states for a vehicle operator based on measurement data from one or more sensing devices configured to measure one or more characteristics associated with the vehicle operator, determining a second value corresponding to a second physiological state of the plurality of different physiological states for the vehicle operator based on output signals from one or more user interface elements, determining an aggregate indicator of a current physiological state of the plurality of different physiological states for the vehicle operator as a function of the first value and the second value using a first weighting factor associated with the first value and a second weighting factor associated with the second value, and automatically initiating a remedial action based on the aggregate indicator. The first weighting factor and the second weighting factor are different and at least one of the first weighting factor and the second weighting factor varies during operation of the vehicle.

In another example, a computer-readable medium having computer-executable instructions stored thereon is provided. When executed by a processing system, the computer-executable instructions cause the processing system to determine a first value corresponding to a first physiological state of a plurality of different physiological states for a vehicle operator based on measurement data from one or more sensing devices configured to measure one or more characteristics associated with the vehicle operator, determine a second value corresponding to a second physiological state of the plurality of different physiological states for the vehicle operator based on output signals from one or more user interface elements, determine an aggregate indicator of a current physiological state of the plurality of different physiological states for the vehicle operator as a function of the first value and the second value using a first weighting factor associated with the first value and a second weighting factor associated with the second value, and automatically initiate a remedial action based on the aggregate indicator.

An aircraft system is also provided. The exemplary aircraft system includes a sensing device to obtain physiological measurement data for a pilot of an aircraft, a user interface element onboard the aircraft to provide signals responsive to the pilot interacting with the user interface element, and a processing system coupled to the sensing device and the user interface element to provide a multimodal monitoring service. The multimodal monitoring services is configurable to determine a biometric indication of a first physiological state of a plurality of different physiological states for the pilot based at least in part on the physiological measurement data, determine an activity-based indication of a second physiological state of the plurality of different physiological states for the pilot based on the signals from the user interface element, determine an aggregate indicator of a current physiological state of the plurality of different physiological states for the pilot as a function of the biometric indication and the activity-based indication using a first weighting factor associated with the biometric indication and a second weighting factor associated with the activity-based indication, and automatically initiate a remedial action based on the aggregate indicator. The first weighting factor and the second weighting factor are different and at least one of the first weighting factor and the second weighting factor varies during operation of the aircraft.

This summary is provided to describe select concepts in a simplified form that are further described in the detailed description. This summary is not intended to identify key or essential features of the claimed subject matter, nor is it intended to be used as an aid in determining the scope of the claimed subject matter.

### BRIEF DESCRIPTION OF THE DRAWINGS

Embodiments of the subject matter will hereinafter be described in conjunction with the following drawing figures, wherein like numerals denote like elements, and:
FIG. 1 is a block diagram illustrating an aircraft system suitable for use with a vehicle such as an aircraft in accordance with one or more exemplary embodiments;
FIG. 2 is a block diagram illustrating multimodal monitoring system suitable for use with the aircraft system of FIG. 1 in accordance with one or more exemplary embodiments;
FIG. 3 is a flow diagram illustrating a biometric state determination process suitable for implementation by the multimodal monitoring system of FIG. 2 in accordance with one or more exemplary embodiments; and
FIG. 4 is a flow diagram illustrating a multimodal monitoring process suitable for implementation by the multimodal monitoring system of FIG. 2 in accordance with one or more exemplary embodiments.

### DETAILED DESCRIPTION

The following detailed description is merely exemplary in nature and is not intended to limit the subject matter of the application and uses thereof. Furthermore, there is no intention to be bound by any theory presented in the preceding background, brief summary, or the following detailed description.

Embodiments of the subject matter described herein generally relate to systems and methods that facilitate autonomously and automatically initiating one or more remedial actions based on the current physiological state of a pilot or other vehicle operator. For example, in situations where there is single pilot operation with no passengers, young passengers, inattentive passengers and/or passengers who are otherwise inexperienced with operating an aircraft, it is desirable to provide a means for activating autonomous assisted landing functionality in an automated manner without potential human error or lapses. Accordingly, the subject matter described herein provides pilot monitoring methods and systems for automatically and autonomously activating assisted landing functionality or initiating other remedial action(s) without requiring involvement of any onboard passengers or other individuals. For example, as described in U.S. Patent Application Serial No. 17/180,954, filed February 22, 2021, which is incorporated by reference herein in its entirety, an aircraft may be equipped with an auto land function or an emergency land function (alternatively referred to herein as autoland functionality) that provides fully autonomous and automatic configuration of the aircraft for landing, and provide automatic flare and centerline guidance to a full stop condition. That said, it should be appreciated the subject matter described herein is not limited to any particular type of remedial action, and in practice, the remedial action(s) can include, but are not limited to, one or more alerts or user notifications for remote or ground support or other supervisory flight control action (e.g., triggering ground control rerouting aircraft around weather due to pilot condition), and/or one or more alerts or user notifications configured to maintain pilot engagement or attention on operating the aircraft (e.g., triggering the pilot to wake up, get a cup of coffee, etc.). For purposes of explanation, the subject matter may be primarily described herein in the context of an aircraft; however, the subject matter described herein is not necessarily limited to aircraft or avionic environments, and in alternative embodiments, may be implemented in an equivalent manner for automobiles or ground operations, vessels or marine operations, or otherwise in the context of other types of vehicles and travel spaces to autonomously operate the vehicle or initiate other remedial actions based on the physiological state of the vehicle operator.

Exemplary embodiments described herein provide multimodal monitoring to more expeditiously activate or otherwise initiate remedial actions, thereby reducing the amount of time between a change in a pilot's physiological state and a corresponding response to the change in physiological state. In this regard, different physiological state indicia are aggregated or otherwise combined using weighting factors that vary during operation of the aircraft or other vehicle (e.g., based on the current phase of flight or operating state, the reliability, integrity or other metrics associated with the underlying data, and/or the like) to provide an aggregate indicator for the current physiological state with higher reliability and lower false alarms (or false positives) for a particular physiological state by combining different physiological state indicia in a learned manner. For example, measurement data from one or more different sensing devices measuring characteristics associated with the pilot or vehicle operator (e.g., heart rate, heart rate variability, acceleration, movement, etc.) may be analyzed to classify the measurement data and provide one or more corresponding biometric indicators of the current physiological state of the pilot from among a plurality of different potential physiological states. In addition to biometric indicia of the current physiological state, the multimodal monitoring processes described herein also monitor the pilot interactions and activity across a variety of different input devices, interface elements and/or other onboard systems to detect, identify or otherwise classify the physiological state of the pilot based on the frequency and/or absence of a pilot interaction over a preceding period of time. The activity-based physiological state indicator is fused, combined or otherwise aggregated with the biometric physiological state indicator(s) using the weighting factors to arrive at an aggregate indictor of the current physiological state of the pilot as a function of the activity-based physiological state indicator(s) and the biometric physiological state indicator(s).

Additionally, in some embodiments, the multimodal monitoring processes described herein also monitor the pilot or other vehicle operator using one or more cameras to visually detect, identify or otherwise classify the physiological state of the pilot based on the visually observed behavior of the pilot. In this regard, a vision-based physiological state indicator may be calculated or otherwise determined based on the observed movements, posture or other visual characteristics of the pilot over a preceding period of time. The vision-based physiological state indicator may be similarly fused, combined or otherwise aggregated with the biometric physiological state indicator(s) and/or activity-based physiological state indicator(s) using the weighting factors to arrive at an aggregate indictor of the current physiological state of the pilot as a function of the activity-based physiological state indicator(s), the biometric physiological state indicator(s) and the vision-based physiological state indicator(s).

When the aggregated indicator corresponds to a particular condition (e.g., a potential incapacitation event) or otherwise satisfies one or more thresholds, one or more notifications or alerts may be provided in a progressively escalating manner to improve the physiological state of the pilot (e.g., to arouse a drowsy pilot) or otherwise encourage pilot interaction before automatically and autonomously activating other automated functionality responsive to the physiological state of the pilot. In this regard, by aggregating and weighting the different physiological state indicators in a manner that varies to account for the current operational context (e.g., the current flight phase, etc.) or other contemporaneous factors (e.g., measurement data integrity, reliability, etc.), the resulting aggregated assessment of the current physiological state achieves higher reliability and results in lower false alarms or false positives. Improving the reliability and reducing the likelihood of false alarms or false positives for particular physiological states allows for alerting thresholds to be reduced or otherwise made more sensitive to changes in the aggregated indicator of the current physiological state, thereby allowing the multimodal monitoring to respond more quickly to a change in the pilot's physiological state. For example, in practice, depending on the current operational context or other contemporaneous conditions, biometric measurements of the physiological state may lead or otherwise indicate a particular physiological state in advance of actual occurrence of that physiological state (e.g., the observed or ground truth physiological state), while activity-based assessments of the physiological state may lag or otherwise follow the actual occurrence of that physiological state, or vice versa. In this regard, the weighting factors for fusing the biometric and activity-based physiological state indica account for the current operational context or other contemporaneous conditions that are likely to influence the respective indicator relative to the probable actual physiological state, thereby resulting in an aggregate assessment of the current physiological state that more accurately tracks or otherwise aligns with the actual physiological state of the pilot.

It will be appreciated that the subject matter described herein is advantageous in numerous different scenarios. For example, in single pilot operation without passengers, if the pilot becomes drowsy or begins to fall asleep and risks becoming incapacitated and unable to land the aircraft while no one else is onboard, the multimodal monitoring process will be able to more detect the drowsiness or change in physiological state to proactively generate alerts to prevent the pilot from falling asleep before the pilot falls asleep. Similarly, in scenarios where the pilot becomes incapacitated and unable to operate the aircraft, the multimodal process will be able to more quickly detect the change in physiological state and responsive thereto activate autoland functionality and/or initiate other remedial actions to expedite the pilot or other individuals onboard the aircraft receiving medical attention and/or providing more time for saving or otherwise restoring normal operation of the aircraft.

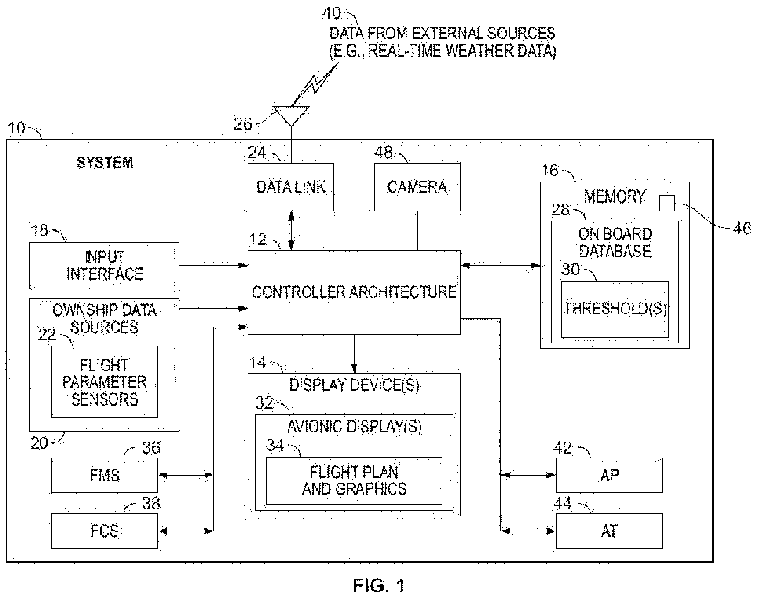

FIG. 1 depicts an exemplary embodiment of an aircraft system 10 suitable for use with an aircraft. As described in greater detail below, in exemplary embodiments, the multimodal pilot monitoring processes described herein are configurable to automatically activate or otherwise initiate one or more remedial actions in an autonomous manner (e.g., without requiring manual engagement by a crew member, passenger, or other individual onboard the aircraft) when a particular physiological state of the pilot is detected or otherwise in response to detecting a change in the current physiological state of the pilot. In some embodiments, the aircraft system 10 autonomously and automatically configures or otherwise operates the aircraft in a manner that is influenced by the current physiological state of the pilot. For example, the aircraft system 10 may automatically initiate autopilot functionality, autoland functionality, or another autonomous operating mode when the current physiological state of the pilot indicates the pilot may be unable or incapable of manually flying the aircraft.

As schematically depicted in FIG. 1, aircraft system 10 includes the following components or subsystems, each of which may assume the form of a single device or multiple interconnected devices: a controller architecture 12, at least one display device 14, computer-readable storage media or memory 16, a pilot input interface 18, an automatic-pilot system (AP) 42 and an automatic throttle system (AT) 44, and an auto-braking system. Aircraft system 10 may further contain ownship data sources 20 including on-board sensors of temperature, humidity, pressure, and the like. In various embodiments, ownship data sources include an array of flight parameter sensors 22. In various embodiments, aircraft system 10 includes a camera 48 oriented in a cockpit to take pictures of the user/pilot.

The aircraft system 10 may be separate from or integrated with: a flight management system (FMS) 36 and a flight control system (FCS) 38. Aircraft system 10 may also contain a datalink subsystem 24 including an antenna 26, which may wirelessly transmit data to and receive data (40) from various sources external to system 10, such as a cloud-based weather (WX) forecasting service of the type discussed below.

Although schematically illustrated in FIG. 1 as a single unit, the individual elements and components of aircraft system 10 can be implemented in a distributed manner utilizing any practical number of physically-distinct and operatively-interconnected pieces of hardware or equipment.

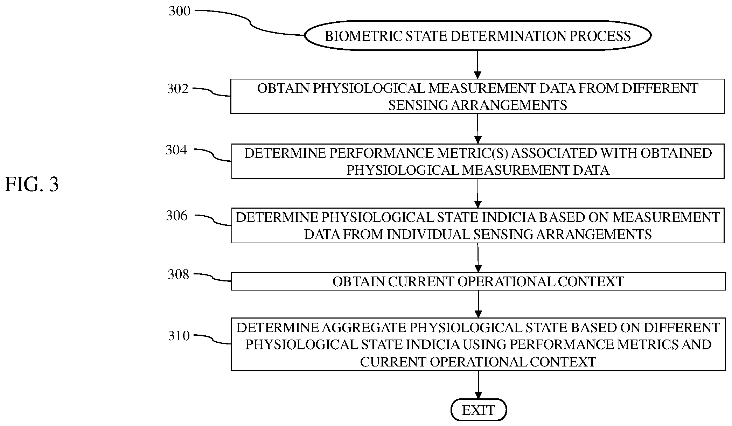

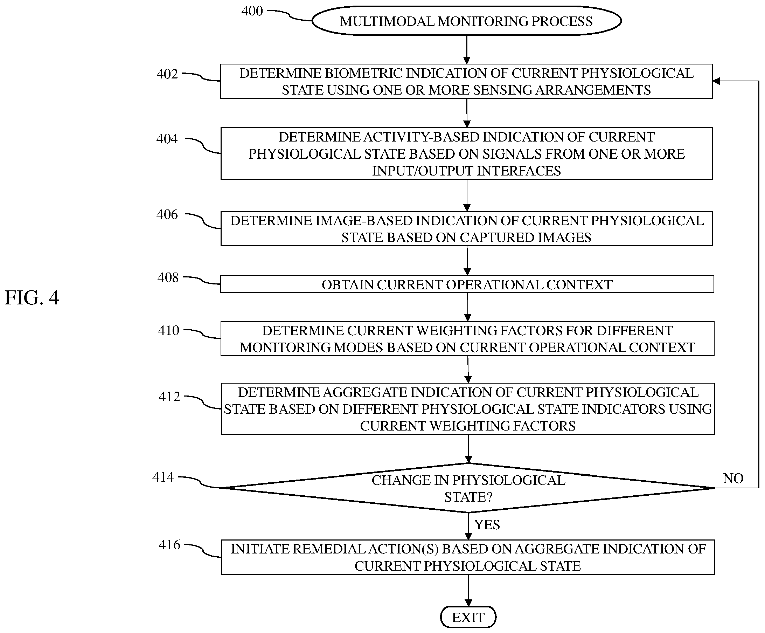

The term "controller architecture," as appearing herein, broadly encompasses those components utilized to carry-out or otherwise support the processing functionalities of aircraft system 10. Accordingly, controller architecture 12 can encompass or may be associated with any number of individual processors, flight control computers, navigational equipment pieces, computer-readable memories (including or in addition to memory 16), power supplies, storage devices, interface cards, and other standardized components. In various embodiments, controller architecture 12 is embodied as an enhanced computer system that includes or cooperates with at least one firmware and software program 46 (generally, computer-readable instructions that embody an algorithm) for carrying-out the various process tasks, calculations, and control/display functions described herein. During operation, the controller architecture 12 may be pre-programmed with, or load and then execute the at least one firmware or software program 46 to thereby perform the various process steps, tasks, calculations, and control/display functions described herein. In one or more exemplary embodiments described herein, the software program 46 implements or otherwise supports the biometric state determination process of FIG. 3 and/or the multimodal monitoring process of FIG. 4 described in greater detail below.

Still referring to FIG. 1, controller architecture 12 may utilize the datalink 24 to exchange data with one or more external sources 40 to support operation of aircraft system 10 in embodiments. In various embodiments, the datalink 24 functionality is integrated within the controller architecture 12. In various embodiments, bidirectional wireless data exchange may occur over a communications network, such as a public or private network implemented in accordance with Transmission Control Protocol/Internet Protocol architectures or other conventional protocol standards. Encryption and mutual authentication techniques may be applied, as appropriate, to ensure data security.

Memory 16 can encompass any number and type of storage media suitable for storing computer-readable code or instructions, such as the aforementioned software program, as well as other data generally supporting the operation of aircraft system 10. In certain embodiments, memory 16 may contain one or more databases 28, such as geographical (terrain), airport features database (providing runways and taxiways), navigational, and historical weather databases, which may be updated on a periodic or iterative basis to ensure data timeliness. The databases maintained in memory 16 may be shared by other systems onboard the aircraft carrying aircraft system 10, such as an Enhanced Ground Proximity Warning System (EGPWS) or a Runway Awareness and Advisory System (RAAS). Memory 16 may also store the software program 46 and/or one or more threshold values, as generically represented by box 30. In various embodiments, the controller architecture 12 has integrated therein suitable memory for processing calculations and for storing the software program 46 and/or the thresholds 30.

Flight parameter sensors 22 supply various types of data or measurements to controller architecture 12 during aircraft flight. In various embodiments, flight parameter sensors 22 provide data and measurements from a Full Authority Digital Engine Control (FADEC), such data or measurements may include engine status (e.g., an engine-out (EO) condition signal) and fuel flow to the engine. In aircraft not having a FADEC, engine status and fuel flow may be determined based on monitored generator current in the engine.

In various embodiments, the flight parameter sensors 22 also supply aircraft status data for the aircraft, including, without limitation: airspeed data, groundspeed data, altitude data, attitude data including pitch data and roll measurements, heading information, flight track data, inertial reference system measurements, Flight Path Angle (FPA) measurements, and yaw data. In various embodiments, aircraft status data for the aircraft also includes one or more of: flight path data, data related to aircraft weight, time/date information, remaining battery time, data related to atmospheric conditions, radar altitude data, geometric altitude data, wind speed and direction data. Further, in certain embodiments of system 10, controller architecture 12 and the other components of aircraft system 10 may be included within or cooperate with any number and type of systems commonly deployed onboard aircraft including, for example, an FMS 36, an Attitude Heading Reference System (AHRS), an Instrument Landing System (ILS), and/or an Inertial Reference System (IRS), to list but a few examples.

With continued reference to FIG. 1, display device 14 can include any number and type of image generating devices and respective display drivers to generate one or more avionic displays. The display device 14 can embody a touch-screen display. When aircraft system 10 is utilized to construct flight plans for a manned aircraft, display device 14 may be affixed to the static structure of the aircraft cockpit as, for example, a Head Down Display (HDD) or Head Up Display (HUD) unit. Alternatively, display device 14 may assume the form of a movable display device (e.g., a pilot-worn display device) or a portable display device, such as an Electronic Flight Bag (EFB), a laptop, or a tablet computer carried into the aircraft cockpit by a pilot.

At least one avionic display 32 is generated on display device 14 during operation of aircraft system 10; the term "avionic display" defined as synonymous with the term "aircraft-related display" and encompassing displays generated in textual, graphical, cartographical, and other formats. Avionic display 32 is generated to include various visual elements or flight plan graphics 34, which may be referenced by a pilot. The graphics 34 can include, for example, textual readouts or text annunciations pertaining to operation of the aircraft and/or the aircraft system 10. The avionic display or displays 32 generated by aircraft system 10 can include alphanumerical input displays of the type commonly presented on the screens of MCDUs, as well as Control Display Units (CDUs) generally. The avionic display or displays 32 generated by aircraft system 10 can also generate various other types of displays on which symbology, text annunciations, and other graphics pertaining to flight planning. Embodiments of aircraft system 10 can generate graphics 34 on one or more two dimensional (2D) avionic displays, such a horizontal or vertical navigation display; and/or on one or more three dimensional (3D) avionic displays, such as a Primary Flight Display (PFD) or an exocentric 3D avionic display. In some embodiments, the display device(s) 14 have integrated therein the necessary drivers and audio devices to additionally provide aural alerts, emitting sounds and speech.

Via various display and graphics systems processes, the graphics 34 on the avionic display or displays 32 can include a displayed button to activate the functions and various alphanumeric messages overlaid on a lateral display or a vertical display. The avionic display or displays 32 generated by aircraft system 10 can also generate various other types of displays on which symbology, text annunciations, and other graphics pertaining to flight planning. Embodiments of aircraft system 10 can generate graphics 34 on one or more two dimensional (2D) avionic displays, such a horizontal or vertical navigation display; and/or on one or more three dimensional (3D) avionic displays, such as a Primary Flight Display (PFD) or an exocentric 3D avionic display.

In various embodiments, a human-machine interface (HMI), such as the above described touch screen display, is implemented as an integration of the user interface 18 and a display device 14. Via various display and graphics systems processes, the controller circuit 12 may command and control the touch screen display generating a variety of graphical user interface (GUI) objects or elements described herein, including, for example, buttons, sliders, and the like, which are used to prompt a user to interact with the HMI to provide user input, and to activate respective functions and provide user feedback, responsive to received user input at the GUI element.

In various embodiments, one or more of the multimodal pilot monitoring process steps described herein are embodied in an algorithm encoded into a software program 46 and executed as computer-implemented functions or process steps, such as, by the controller architecture 12. In some embodiments, the process steps are aggregated into larger process blocks, and the controller architecture 12 directs or delegates the aggregated larger process blocks to various systems on-board the aircraft to perform. In exemplary embodiments, the controller architecture 12 activates autonomous functionality and/or otherwise initiates one or more remedial actions responsive to the multimodal pilot monitoring process determining a remedial action should be initiated based on the aggregated assessment of the current physiological state of the pilot, as described in greater detail below. In some embodiments, the cockpit camera 48 may be utilized to verify or otherwise confirm the physiological state or condition detected by the multimodal pilot monitoring process, for example, by obtaining an eyelid position or pupil dilation input, via a cockpit camera 48, and processing this input with visual algorithms included in program 46 to classify the visually observed physiological state of the pilot. Responsive to the detection of a particular physiological state, in some embodiments, the controller architecture 12 may generate a prompt for the pilot to manually respond or interact with the system 10 to cancel an impending automatic activation of autoland functionality or another autonomous operating mode prior to the automatic activation. In one embodiment, the prompt is a GUI object with a timer countdown that is displayed while counting down.

In various embodiments, responsive to automated activation of the autoland functionality or another autonomous operating mode, the FCS 38 automatically activates the AT 44 and AP 42 functions, and the controller architecture 12 begins commanding the AP 42 and AT 44 to autonomously operate the aircraft in accordance with the desired autonomous functionality for the current physiological state of the pilot. In exemplary embodiments, responsive to activation of the autonomous functionality, the controller architecture 12 automatically generates a flight plan for autonomously operating the aircraft, for example, by processing inputs such as terrain, obstacles, weather, aircraft-specific approach capabilities, runway lengths, range, on-ground weather conditions, etc. In some embodiments, the controller architecture 12 generates commands for leveling the aircraft while the flight plan is being updated prior to actively controlling the AP 42 and AT 46 to autonomously operating the aircraft in accordance with the resulting flight plan. For example, the controller architecture 12 may command the FCS 38 to activate a flight director lateral mode (annunciated to the crew as ROL) which commands a wings level lateral command, this may also be referred to as ROL (WNG_LVL) and activate flight path angle (FPA) with a target FPA of 0 degrees to level the aircraft and await FMS flight plan activation. When generating the flight plan, the controller architecture 12 may interface with an instrument navigation (INAV) onboard terrain/obstacle database to provide terrain awareness and/or interface with the INAV weather (WX) layers to determine en route weather.

In various embodiments, responsive to activation of the autonomous functionality, the controller architecture 12 may select a different airport from a previously selected airport for landing the aircraft. For example, when the current physiological state indicates speed is a priority (e.g., an incapacitated pilot) and a different airport provides a quicker option, the controller architecture 12 may autonomously and automatically select a nearest suitable airport and an associated route thereto, and then autonomously control the AP 42 and AT 44 to fly the aircraft along the route to a final approach fix before autonomously communicating with air traffic control (ATC), autonomously configuring the aircraft for landing and autonomously landing the aircraft at the nearest suitable airport. In various embodiments, the controller architecture 12 will use GPS altitude for approach calculations when it determines that it cannot be ensured the correct barometric setting has been received. In various embodiments where ILS approach is optimal selection, the controller architecture 12 will automatically tune the NAV radios to the LOC frequency. In various embodiments when LNAV/VNAV becomes active, the controller architecture 12 manages the speed. In the computation of landing performance data, the controller architecture 12 may interface with various third-party off-board products which assist in the automated acquisition of this data, such as Go-direct. Alternatively, in various embodiments, the controller architecture 12 may utilize onboard products, such as satellite weather (SiriusXM) or upgraded ADS-B technology like FIS-B (Flight Information System Broadcast) that require various landing performance data (runway length, winds, temp, etc.) to be entered in to compute the various landing speeds and landing lengths. If the pilot is incapacitated, this cannot be entered, but there are various services the AC may subscribe to (The automatic flight planning service from Go-Direct) which could send digital uplinks to the aircraft to automatically enter this information into the FMS in lieu of pilot. Advantageously, getting this real-time information, rather than just using a 'worst case' assumption, increases the number of runways the controller architecture 12 could pick because it does not have to throw out possible runways to only include the worst-case acceptable runways. In other embodiments, the algorithm executed by the controller architecture 12 picks an approach and landing airport that has a runway large enough to land the aircraft with a built-in safety-factor, regardless of landing performance data. During execution of the flight plan, the controller architecture 12 autonomously controls the aircraft configuration at appropriate points along the flight plan (e.g., flap deployment at appropriate points along the approach profile, gear deployment at appropriate points along the approach profile, and/or the like).

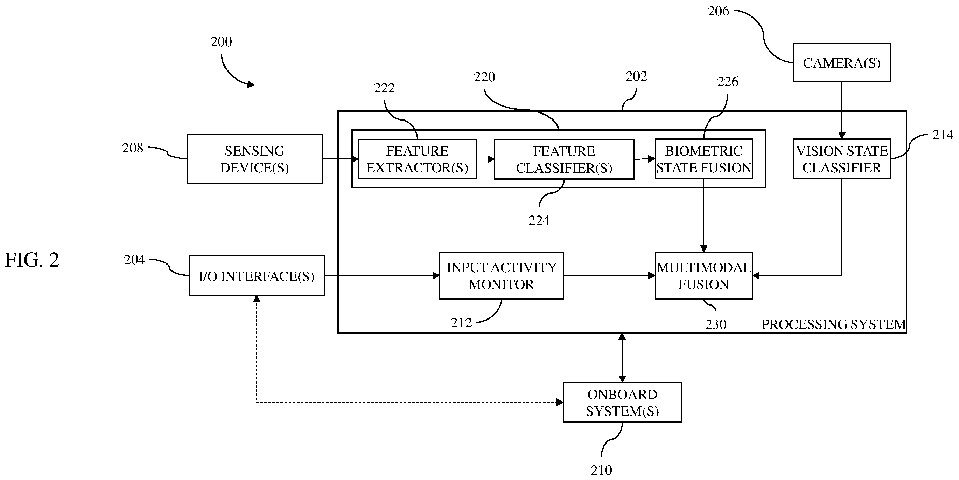

FIG. 2 depicts an exemplary embodiment of a multimodal monitoring system 200 suitable for implementation in connection with an aircraft or other vehicle to provide multimodal monitoring of the physiological state of the pilot or other vehicle operator. For example, the multimodal monitoring system 200 may be incorporated with or otherwise implemented in connection with the aircraft system 10 to support autonomously operating the autopilot system 42 and/or the autothrottle system 44 or initiating other remedial actions responsive to a change in the physiological state of a pilot. It should be appreciated that FIG. 2 merely depicts one simplified representation of the multimodal monitoring system 200 for purposes of explanation and is not intended to be exhaustive or limiting.

The multimodal monitoring system 200 includes a processing system 202 (e.g., controller architecture 12) that is coupled to various different devices 204, 206, 208 that are located onboard the aircraft or otherwise arranged, positioned or configured to monitor a pilot or other operator onboard the aircraft. In exemplary embodiments, the processing system 202 is coupled to one or more input/output (I/O) interface devices 204 (e.g., input user interfaces 18), which generally represent the user input devices that allow a user (e.g., a pilot, co-pilot, or crew member) to interact with a display device (e.g., display device 14) or other onboard systems 210 (e.g., the FMS 36, the FCS 38, the AP 42, the AT 44, etc.). Depending on the embodiment, the I/O interface device(s) 204 may include or otherwise be realized as a keypad, touchpad, keyboard, mouse, touch panel (or touchscreen), joystick, knob, line select key or another suitable device adapted to receive a physical or tactile input from a user, and/or an audio input device, such as a microphone, audio transducer, audio sensor, or the like, that is adapted to allow a user to provide audio input (e.g., in a "hands free" manner). As described in greater detail below, in exemplary embodiments, the processing system 202 is configured to generate, execute or otherwise implement an input activity monitoring service 212 (or input activity monitor) that calculates or otherwise determines a value indicative of a current physiological state of the pilot based on signals received from the I/O interfaces 204 that are indicative of the pilot interactions with one or more onboard systems 210.

In exemplary embodiments, the processing system 202 is also coupled to one or more cameras 206 (e.g., camera 48) onboard the aircraft that are positioned or otherwise oriented within the cockpit of the aircraft to capture pictures, video and/or other imagery of the pilot or other users within the cockpit of the aircraft. As described in greater detail below, in exemplary embodiments, the processing system 202 is configured to generate, execute or otherwise implement a vision state classification service 214 (or vision state classifier) that calculates or otherwise determines a value indicative of a current physiological state of the pilot based on image data received from the onboard camera(s) 206 that are indicative of the observed visual state of the pilot.

Still referring to FIG. 2, the processing system 202 is also coupled to one or more sensing devices 208 that are configured to measure one or more physiological characteristics of the pilot or other vehicle operator. In this regard, in some embodiments, one or more of the sensing devices 208 may be realized as a wearable sensing device or other device that is associated with or otherwise on the body of the pilot and communicatively coupled to the processing system 202 (e.g., via a wireless communications network). Additionally, or alternatively, one or more of the sensing devices 208 may be located onboard the aircraft, physically integrated with or otherwise associated with the aircraft. For example, a sensing device 208 may be integrated or otherwise incorporated with an I/O interface 204, a seat, or another element within the cockpit of the aircraft. Non-limiting examples of sensing devices 208 that may be utilized in connection with the multimodal monitoring system 200 include, but are not limited to, accelerometers, motion sensors, weight sensors, pressure sensors, temperature sensors, heart rate sensors, pulse sensors, oxygen sensors, optical or magnetic position tracking devices, or any other sort of sensor capable of obtaining physiological measurement data pertaining to the physiological condition or state of the pilot or other vehicle operator.

As described in greater detail below, in one or more exemplary implementations, the processing system 202 is configured to support, execute or otherwise implement a biometric state classification service 220 (or biometric state classifier) that receives one or more streams of physiological measurement data from the one or more sensing device(s) 208 and calculates or otherwise determines an aggregate biometric indicator of the current physiological state of the pilot as a function of the different streams of physiological measurement data. For example, in the illustrated embodiment, the biometric state classifier 220 includes, for each different stream of physiological measurement data, a corresponding feature extraction engine 222 (or feature extractor) that receives or otherwise obtains the raw measurement data samples from the respective sensing device 208 and calculates, extracts or otherwise determines one or more metrics, statistics or features of the physiological measurement data that are correlative to or otherwise indicative of a particular physiological state from among the plurality of different potential physiological states for the pilot. For each respective physiological measurement data stream, the extracted physiological measurement features determined by the respective feature extractor 222 are input or otherwise provided to a corresponding feature classification engine 224 (or feature classifier) that maps or otherwise determines a probable physiological state of the plurality of different potential physiological states for the pilot as a function of the sequence of extracted physiological measurement features input to the respective feature classifier 224. In this regard, the feature classifier 224 classifies the pilot's current physiological state into one of the plurality of different potential physiological states based on the time-varying sequence of extracted measurement features from a respective sensing device 208.

For example, a heart rate sensing arrangement 208 may output or otherwise provide a stream of heart rate measurement data samples to a corresponding heart rate feature extractor 222, which, in turn analyzes the sequence of heart rate measurement data samples over time to calculate, extract or otherwise determine current values for one or more heart rate metrics (e.g., average heart rate, heart rate variability, and/or the like) that are correlative to or otherwise indicative of a particular physiological state. The extracted heart rate features output by the heart rate feature extractor 222 are then input or otherwise provided to the heart rate feature classifier 224 that analyzes the values of the extracted heart rate features over time to classify or otherwise characterize the current physiological state of the pilot based on the values for the extracted heart rate feature(s) and outputs a corresponding heart rate-based indicator of the current physiological state. In a similar manner, an acceleration sensing arrangement 208 may output or otherwise provide a stream of acceleration measurement data samples to a corresponding acceleration feature extractor 222, which, in turn analyzes the sequence of acceleration measurement data samples over time to calculate, extract or otherwise determine current values for one or more acceleration metrics or features that are correlative to or otherwise indicative of a particular physiological state. The extracted acceleration features output by the acceleration feature extractor 222 are then input or otherwise provided to the acceleration feature classifier 224 that analyzes the values of the extracted acceleration features over time to classify or otherwise characterize the current physiological state of the pilot based on the values for the extracted acceleration feature(s) and outputs a corresponding acceleration-based indicator of the current physiological state. Such acceleration feature extraction can ideally distinguish accelerations resulted from a platform and operators on board of the vehicle.

In one or more embodiments, the feature extractors 222 and/or the feature classifiers 224 are derived using machine learning or other artificial intelligence techniques to analyze historical relationships between different metrics, statistics or other features of a respective physiological measurement data stream to identify the subset of features to be extracted that are correlative to or otherwise indicative of a particular physiological state from among the plurality of different potential physiological states for the pilot, as well as how the occurrence and/or sequence of different combinations of extracted features with respect to time should be mapped, converted or otherwise classified as a particular one of the different potential physiological states. In practice, some embodiments of the feature extractors 222 and/or the feature classifiers 224 may be configured to receive status information indicative of the current operational context associated with the aircraft from one or more onboard systems 210 to dynamically adjust the relative weighting or importance of different measurement data features with respect to time depending on the current operational context based on historical relationships between those features and the actual observed physiological state of a pilot with respect to the contemporaneous operational context of the aircraft, as described in greater detail below. Similarly, the algorithms or models for the feature extractors 222 and/or the feature classifiers 224 may be trained using, as input variables, one or more performance metrics associated with the respective sensing device 208 and/or the respective physiological measurement data stream to dynamically adjust the relative weighting or importance of different measurement data features with respect to time depending on the current performance metric(s) based on historical relationships between those features and the actual observed physiological state of a pilot with respect to the contemporaneous performance metrics. When the operation environment is more dynamic, for example, within more turbulent weather conditions, the weighing factor may be adjusted in real-time to favor more toward aircraft system parameters due to potentially higher uncertainty in physiological parameter measurements.

Still referring to FIG. 2, the illustrated embodiment of the biometric state classifier 220 includes a biometric state fusion engine 226 that calculates or otherwise determines an aggregate biometric indicator of the current physiological state as a function of the different physiological state indicia associated with the different streams of physiological measurement data. For example, the biometric state fusion engine 226 may map a particular combination and/or sequence of heart rate-based physiological state indicia from the heart rate feature classifier 224 and the acceleration-based physiological state indicia from the acceleration feature classifier 224 to a corresponding aggregate value indicative of the physiological state. In this regard, the biometric state fusion engine 226 may utilize a biometric state fusion model derived using machine learning or other artificial intelligence techniques to develop an equation or formula for calculating a probable value for the physiological state of the pilot or other vehicle operator as a function of input physiological state indicia associated with different types of sensing devices 208 and/or different streams of physiological measurement data based on historical relationships between the respective physiological characteristics being measured from pilots or other operators during prior instances of vehicle operation and observed physiological states of those pilots or operators during those prior instances of vehicle operation. As a result, the biometric state fusion engine 226 may assign different weighting factors to different types of sensing devices 208 and/or different streams of physiological measurement data based on the relative strength or degree of correlation between the physiological state indicia output by the feature classifier 224 for the respective sensing device 208 and the observed physiological states. Thus, particular sensing devices 208 or physiological measurements that are more predictive of the actual physiological state may be more heavily weighted, while sensing devices 208 or physiological measurements that are less predictive of the actual physiological state may be less heavily weighted.

In one or more embodiments, the model utilized by the biometric state fusion engine 226 is trained or otherwise developed using contextual operational variables as inputs to the model, such that the relative weightings assigned to the different types of sensing devices 208 and/or different streams of physiological measurement data may vary depending on the current operational context of the aircraft. For example, the biometric state fusion model may be trained in a manner that accounts for historical relationships between the current phase of flight, the current aircraft altitude, the current aircraft configuration and/or the like, the observed physiological pilot states during those respective operational contexts, and the corresponding historical physiological measurement data output by the different types of sensing devices 208 during those respective operational contexts. Thus, particular sensing devices 208 or physiological measurements that are more predictive of the actual physiological state for some flight phases or operational contexts may be more heavily weighted while the aircraft is currently operating in that particular flight phase or operational context, while those same sensing devices 208 or physiological measurements may be less heavily weighted in other flight phases or operational contexts where different types of sensing devices 208 or physiological measurements are more correlative to the pilot's physiological state.

In one or more exemplary embodiments, the feature extractor 222 calculates or otherwise determines one or more performance metrics associated with the respective sensor device 208 and/or the respective physiological measurement data stream, which, in turn, may be utilized by the downstream feature classifier 224 and/or biometric state fusion engine 226 to characterize the physiological state in a manner that is influenced by the performance metrics associated with the respective sensor device 208 and/or the respective physiological measurement data stream. For example, the feature extractor 222 may calculate or otherwise determine one or more statistics or other performance metrics that characterize one or more of the accuracy, reliability, integrity, stability and/or quality of the measurement data samples provided by a respective sensor device 208. In this regard, as the performance metrics associated with a particular sensor device 208 and/or physiological measurement data stream change over time, the relative weight or influence given to changes or fluctuations in that physiological measurement data may increase and/or decrease over time to reflect the quality of the underlying physiological measurement data. For example, at the beginning of a flight, when relatively few physiological measurement data samples are available, classifying the physiological state of a pilot or otherwise identifying a change in the physiological state based on those physiological measurement data samples (or trends thereof) may be less reliable than later in the flight when a greater number of physiological measurement data samples allow for interferences to be drawn with greater precision and accuracy. Thus, the feature classifier 224 may assign different weights to different features in a manner that varies over time to reflect the probative value of the particular feature with respect to the actual or likely current physiological state of the pilot based on the preceding physiological measurement data samples. Similarly, based on the performance metrics associated with the different sensing devices 208 and/or different physiological measurement data streams, the biometric state fusion engine 226 may dynamically vary the weightings assigned to the different physiological state indicia associated with the different streams of physiological measurement data based on the relative quality of the underlying physiological measurement data.

Still referring to FIG. 2, in exemplary embodiments, the processing system 202 is also configured to support, execute or otherwise implement a multimodal fusion service 230 that receives different indicia of the current physiological state of the pilot or vehicle operator from the different pilot monitoring services 212, 214, 220 and calculates or otherwise determines an aggregate indicator of the current physiological state of the pilot as a function of the different physiological state indicia derived using different devices 204, 206, 208 and/or different underlying data. In this regard, the multimodal fusion service 230 may calculate or otherwise determine a current value for the current physiological state of a pilot as a weighted function of the aggregate biometric physiological state indicia output by the biometric state classifier 220, the vision-based physiological state indicia output by the vision state classifier 214 and the activity-based physiological state indicia output by the input activity monitor 212. In a similar manner as described above in the context of the biometric state fusion engine 226, the multimodal fusion service 230 may utilize a multimodal physiological state fusion model derived using machine learning or other artificial intelligence techniques to develop an equation or formula for calculating a probable value for the physiological state of the pilot or other vehicle operator as a function of input physiological state indicia associated with different modes of monitoring based on historical relationships between the respective physiological state indicia during prior instances of vehicle operation and observed physiological states of those pilots or operators during those prior instances of vehicle operation. As a result, the multimodal fusion service 230 may assign different weighting factors to the different physiological state indicia based on the relative strength or degree of correlation between the physiological state indicia output by a respective pilot monitoring service 212, 214, 220 and the observed physiological states. Thus, a particular mode of pilot monitoring that is more predictive of the actual physiological state may be more heavily weighted, while other modes of pilot monitoring that are less predictive of the actual physiological state may be less heavily weighted.

Additionally, the multimodal fusion service 230 may account for the current operational context of the aircraft, such that the relative weightings assigned to the different physiological state indicia may vary depending on the current operational context of the aircraft. For example, the multimodal physiological state fusion model may be trained in a manner that accounts for historical relationships between the current phase of flight, the current aircraft altitude, the current aircraft configuration and/or the like, the observed physiological pilot states during those respective operational contexts, and the corresponding historical physiological state indicia output by the different pilot monitoring services 212, 214, 220 during those respective operational contexts. Thus, a particular mode of pilot monitoring that are more predictive of the actual physiological state for some flight phases or operational contexts may be more heavily weighted while the aircraft is currently operating in that particular flight phase or operational context, while the same mode of pilot monitoring may be less heavily weighted in other flight phases or operational contexts where different modes of pilot monitoring are more predictive of the pilot's actual, real-time physiological state.

In a similar manner as described above in the context of the biometric state fusion engine 226, in some implementations, the multimodal fusion service 230 may also account for performance metrics associated with the respective sensor devices 208 and/or the respective physiological measurement data streams with respect to time. For example, earlier in the flight when there are fewer physiological measurement data samples, the multimodal fusion service 230 may decrease the relative weighting assigned to a biometric physiological state indicator provided by the biometric state classifier 220 but progressively increase the relative weighting assigned to the biometric physiological state indicator over time as the inferences drawn from the physiological measurement data become more reliable due to the increased number of underlying physiological measurement data samples. In this regard, the multimodal physiological state fusion model may be trained in a manner that accounts for historical relationships between the aircraft operational context, the observed physiological pilot states during those respective aircraft operational contexts, the corresponding historical physiological state indicia output by the different pilot monitoring services 212, 214, 220 during those respective operational contexts, and performance metrics associated with the different pilot monitoring services 212, 214, 220 (or the underlying data upon which a respective pilot monitoring service 212, 214, 220 determines the physiological state). Thus, the weighting factors assigned to the different physiological state indicia output by the different pilot monitoring services 212, 214, 220 may vary with respect to the current flight phase or other operational context associated with the aircraft (e.g., when there is a change in flight phase or operational context), while also varying with respect to time while the operational context is maintained the same based on dynamic changes to one or more performance metrics associated with (or quality of) the underlying data upon which a respective physiological state indicator is based and historical correlations between the performance metric(s) and the accuracy or reliability of the respective physiological state indicator.

It should be appreciated that although FIG. 2 depicts the biometric state fusion engine 226 and the multimodal fusion service 230 as separate and distinct fusion stages, in alternative embodiments, the biometric state fusion and the multimodal fusion may be integrated or otherwise combined in a single fusion stage. In such embodiments, the different physiological state indicia associated with different types of sensing devices 208 and different streams of physiological measurement data may be fused or otherwise combined with the activity-based and image-based physiological state indicia at a single stage that accounts for the historical relationships and correlations between the respective physiological state indicia and the flight phase, configuration and/or other operational context of the aircraft. In other words, the aggregate indicator of the physiological state may be determined as a function of the activity-based physiological state indicator, the vision-based indicator, and the different physiological state indicators associated with the different sensing devices 208 using weighting factors associated with the respective indicators, where the weighting factors dynamically vary relative to one another to reflect the current aircraft flight phase and/or other real-time operational contextual information associated with the aircraft.

Based on the aggregate physiological state indicator determined by the multimodal fusion service 230, the processing system 202 may interact with one or more onboard systems 210 to autonomously and automatically initiate one or more remedial actions based on the current value for the aggregate physiological state indicator. For example, the processing system 202 may generate one or more graphical notifications or alerts on one or more display devices onboard the aircraft to prompt the pilot to acknowledge the alerts or otherwise perform some action, thereby influencing the pilot's physiological state. Additionally, in some embodiments, the processing system 202 may generate auditory alerts in concert with graphical notifications. In this manner, the processing system 202 may initiate remedial actions designed to alter or reverse the progression of the pilot's physiological state, for example, to prevent drowsiness from progressing to sleep or some other incapacitated state. That said, when the aggregate physiological state indicator output by the multimodal fusion service 230 indicates that the pilot is incapacitated or otherwise unable to operate the aircraft, the processing system 202 may interact with the FMS 36, the autopilot 42 and/or the autothrottle 44 to autonomously operate the aircraft and/or initiate autoland functionality.

Still referring to FIG. 2, the processing system 202 generally represents a computing device, computing system or another combination of processing logic, circuitry, hardware, and/or other components configured to support the processes, tasks, operations, and/or functions described herein. In this regard, the processing system 202 may be implemented using any suitable processing system and/or device, such as, for example, one or more processors, central processing units (CPUs), controllers, microprocessors, microcontrollers, processing cores and/or other hardware computing resources configured to support the operation of the processing system 202 described herein. The processing system 202 may also include or otherwise access a data storage element (or memory) capable of storing programming instructions for execution by the processing system 202, that, when read and executed, cause processing system 202 to support the processes described herein. Depending on the implementation, the memory may be realized as a random-access memory (RAM), read only memory (ROM), flash memory, magnetic or optical mass storage, or any other suitable non-transitory short or long-term data storage or other computer-readable media, and/or any suitable combination thereof. In one or more implementations, the programming instructions cause the processing system 202 to create, generate, or otherwise facilitate the various pilot monitoring services 212, 214, 220 that support or otherwise facilitate the processes, tasks, operations, and/or functions described herein.

Referring now to FIG. 3, in one or more exemplary embodiments, a multimodal monitoring system associated with an aircraft is configured to support a biometric state determination process 300 to analyze different streams of physiological measurement data and determine a corresponding biometric indicator of the current physiological state of a pilot and perform additional tasks, functions, and operations described below. In this manner, the biometric state determination process 300 maps a particular combination and/or sequence of physiological measurement data streams to a particular physiological state from among a plurality of potential different physiological states. The various tasks performed in connection with the illustrated process 300 may be implemented using hardware, firmware, software executed by processing circuitry, or any combination thereof. For illustrative purposes, the following description may refer to elements mentioned above in connection with FIGS. 1-2. In practice, portions of the biometric state determination process 300 may be performed by different elements of the aircraft system 10 and/or the multimodal monitoring system 200; however, for purposes of explanation, the biometric state determination process 300 may be described herein primarily in the context of the biometric state classifier 220 supported by the processing system 202. It should be appreciated that the biometric state determination process 300 may include any number of additional or alternative tasks, the tasks need not be performed in the illustrated order and/or the tasks may be performed concurrently, and/or the biometric state determination process 300 may be incorporated into a more comprehensive procedure or process having additional functionality not described in detail herein. Moreover, one or more of the tasks shown and described in the context of FIG. 3 could be omitted from a practical embodiment of the biometric state determination process 300 as long as the intended overall functionality remains intact.

Referring to FIG. 3, with continued reference to FIG. 2, the biometric state determination process 300 receives or otherwise obtains measurement data indicative of one or more physiological characteristics of a pilot from one or more different sensing arrangements associated with the pilot and calculates or otherwise determines one or more performance metrics associated with different streams of physiological measurement data from the different sensing arrangements (tasks 302, 304). For example, as described above in the context of FIG. 2, in one or more exemplary implementations, the processing system 202 and/or the biometric state classifier 220 receives or otherwise obtains a stream of physiological measurement data samples from any number of different sensing devices 208 that are configured to measure different physiological characteristics of the pilot, such as, for example, heart rate sensors, acceleration sensors or other motion sensors, oxygen sensors, and/or the like that may be worn on the body of the pilot or otherwise arranged in the cockpit of the aircraft in a manner allows measurement of the respective physiological characteristic of the pilot. The biometric state classifier 220 and/or the feature extractors 222 at the processing system 202 analyze the different streams of physiological measurement data samples to extract, identify or otherwise determine different features of the respective physiological measurement data stream that are correlative to or otherwise probative of the physiological state of the pilot as well as calculating or otherwise determining one or more performance metrics associated with the respective physiological measurement data stream that are indicative of the accuracy or reliability of the respective physiological measurement data stream.

The illustrated biometric state determination process 300 continues by calculating or otherwise determining an indication of the current physiological state of the pilot from among a plurality of potential predefined physiological states for each of the different streams of physiological measurement data from the different sensing arrangements (task 306). For example, as described above in the context of FIG. 2, in exemplary embodiments, a biometric state classifier 220 includes an instance of a physiological measurement feature classifier 224 associated with each different sensing device 208 and physiological measurement data stream that analyzes the respective physiological measurement data sample(s), the feature(s) extracted from the respective physiological measurement data samples and/or the performance metric(s) associated with the respective physiological measurement data stream to map or otherwise convert that input information to a corresponding output indicator that classifies, estimates or otherwise predicts a probable or likely physiological state for the pilot based on the input information derived from the respective physiological measurement data stream. In practice, the plurality of potential predefined physiological states may correspond to different, physiologically distinct levels of alertness, awareness and/or drowsiness that are capable of being exhibited by a human during operation of a vehicle. Thus, the biometric state determination process 300 may determine a first classification of a pilot's physiological state based on information derived from a stream of heart rate measurements for the pilot, a second classification of the pilot's physiological state based on information derived from a stream of acceleration measurements for the pilot, and so on.

Still referring to FIG. 3, in exemplary embodiments, the biometric state determination process 300 identifies or otherwise obtains current status information indicative of the current, real-time operational context associated with the aircraft and then calculates or otherwise determines an aggregate indicator of the current physiological state of the pilot from among the plurality of potential predefined physiological states in a manner that accounts for the current operational context and the performance metrics associated with the different streams of physiological measurement data from the different sensing arrangements (tasks 308, 310). For example, as described above in the context of FIG. 2, the processing system 202 and/or the biometric state classifier 220 may obtain information indicative of the current flight phase, the current aircraft configuration, the current aircraft altitude and/or the like from one or more onboard systems 210, which, in turn, may be utilized by the biometric state fusion engine 226 to assign different weights to the different physiological state indicia associated with the different sensing devices 208 based on the historical correlation or relationship between the expected physiological state assigned based on the respective physiological measurement data stream associated with the respective sensing device 208 and the observed actual contemporaneous physiological state. Additionally, the relative weightings may be further adjusted based on the historical correlation or relationship between the performance metrics associated with the different physiological measurement data streams and the accuracy, reliability and/or probability of the physiological state assigned based on the respective physiological measurement data stream corresponding to the observed actual contemporaneous physiological state. In this manner, the biometric state determination process 300 may determine a context-sensitive aggregated biometric physiological state indicator using multiple different types of sensing devices 208.

Referring now to FIG. 4, in one or more exemplary embodiments, a multimodal monitoring system associated with an aircraft is configured to support a multimodal monitoring process 400 to assess the current physiological state of a pilot and perform additional tasks, functions, and operations described below. The various tasks performed in connection with the illustrated process 400 may be implemented using hardware, firmware, software executed by processing circuitry, or any combination thereof. For illustrative purposes, the following description may refer to elements mentioned above in connection with FIGS. 1-2. In practice, portions of the multimodal monitoring process 400 may be performed by different elements of the aircraft system 10 and/or the multimodal monitoring system 200; however, for purposes of explanation, the multimodal monitoring process 400 may be described herein primarily in the context of the multimodal fusion service 230 supported by the processing system 202. It should be appreciated that the multimodal monitoring process 400 may include any number of additional or alternative tasks, the tasks need not be performed in the illustrated order and/or the tasks may be performed concurrently, and/or the multimodal monitoring process 400 may be incorporated into a more comprehensive procedure or process having additional functionality not described in detail herein. Moreover, one or more of the tasks shown and described in the context of FIG. 4 could be omitted from a practical embodiment of the multimodal monitoring process 400 as long as the intended overall functionality remains intact.

Referring to FIG. 4, with continued reference to FIGS. 1-3, the multimodal monitoring process 400 obtains, identifies or otherwise determines a biometric indication of the current physiological state of pilot using one or more different sensing arrangements (task 402). For example, as described above in the context of FIGS. 2-3, in exemplary embodiments, the multimodal fusion service 230 may receive a value output by the biometric state classifier 220 that represents the estimated or predicted physiological state of the pilot based on the physiological measurement data obtained from the sensing devices 208 configured to measure physiological characteristics associated with the pilot determined in accordance with the biometric state determination process 300.

The multimodal monitoring process 400 also obtains, identifies or otherwise determines an activity-based indication of the current physiological state of pilot based on output signals (or the absence thereof) from one or more input/output user interfaces (task 404). For example, based on the number and/or frequency of signals indicative of user interaction with the I/O user interface devices 204 onboard the aircraft, the input activity monitor 212 may calculate or otherwise determine value that represents the estimated or predicted physiological state of the pilot based on interactions with the I/O devices 204. In a similar manner as described above in the context of the biometric state classifier 220, in some embodiments, the input activity monitor 212 may utilize one or more models derived using machine learning or other artificial intelligence techniques to develop an equation or formula for calculating a probable value for the physiological state of the pilot or other vehicle operator as a function of the observed signals output by the I/O interfaces 204 based on historical relationships between the observed signals output by the I/O interfaces 204 from other pilots or other operators during prior instances of vehicle operation and observed physiological states of those pilots or operators during those prior instances of vehicle operation. In this regard, the input activity monitor 212 may also be configured to account for the current operational context or other performance metrics associated with the signals output by the I/O interfaces 204. In other embodiments, rule-based logic may be utilized to classify or otherwise categorize the physiological state based on the number and/or frequency of user interactions over one or more preceding monitoring periods, which may vary depending on the current flight phase or other operational context. Some examples of activity-based monitoring using monitoring periods are described in U.S. Patent Application Serial No. 17/411,957.

In the illustrated embodiment of FIG. 4, the multimodal monitoring process 400 also obtains, identifies or otherwise determines an image-based indication of the current physiological state of pilot based on analysis of captured images from one or more imaging devices onboard the aircraft (task 406). For example, the vision state classifier 214 may analyze images captured by the camera(s) 206 onboard the aircraft to detect pilot motion, analyze the posture of the pilot, and/or the like, and based on the physical observations of the pilot derived from the captured images, calculate or otherwise determine value that represents the estimated or predicted physiological state of the pilot based on the visual, physical observations of the pilot. In a similar manner as described above, in some embodiments, the vision state classifier 214 may utilize one or more models derived using machine learning or other artificial intelligence techniques to develop an equation or formula for calculating a probable value for the physiological state of the pilot or other vehicle operator as a function of the observed motion, posture or other physical characteristics of the pilot based on historical relationships between the visual observations from other pilots or other operators during prior instances of vehicle operation and observed physiological states of those pilots or operators during those prior instances of vehicle operation. In this regard, the vision state classifier 214 may also be configured to account for the current operational context or other performance metrics associated with the vision-based classification of the physiological state.

Still referring to FIG. 4, in exemplary embodiments, the multimodal monitoring process 400 identifies or otherwise obtains current status information indicative of the current, real-time operational context associated with the aircraft, identifies or otherwise determines different weighting factors to be assigned to the different modes of monitoring the pilot based at least in part on the current operational context, and then calculates or otherwise determines an aggregate indication of the current physiological state of the pilot in a manner that accounts for the current operational context based on the physiological state indicia from the different monitoring modes using the different weighting factors assigned to the different pilot monitoring modes (tasks 408, 410, 412). For example, as described above in the context of FIGS. 2-3, the processing system 202 and/or the multimodal fusion service 230 may obtain information indicative of the current flight phase, the current aircraft configuration, the current aircraft altitude and/or the like from one or more onboard systems 210, which, in turn, may be utilized by the multimodal fusion service 230 to assign different weights to the different physiological state indicia output by the different pilot monitoring services 212, 214, 220 based on the historical correlation or relationship between the expected physiological state output by the respective pilot monitoring service 212, 214, 220 and the previously observed actual (or ground truth) contemporaneous physiological state with respect to the contemporaneous operational context during previous instances of monitoring pilots operating aircraft. In this regard, the weighting factor assigned to a particular monitoring mode may dynamically vary during flight to reflect the relative accuracy or reliability of that particular mode of monitoring the pilot for the current, real-time operational context of the aircraft.

For example, the weighting factor assigned to the activity-based indicator output by the input activity monitor 212 and/or the weighting factor assigned to the vision-based indicator output by the vision state classifier 214 may be increased during aircraft operation in flight phases where pilots historically exhibit a higher workload or increased interaction with avionics or other onboard systems (e.g., takeoff, approach, etc.), while being decreased during aircraft operation in flight phases where pilots historically exhibit a reduced workload or decreased interaction with avionics or other onboard systems (e.g., cruise, etc.). Similarly, the weighting factor assigned to the biometric indicator output by the biometric state classifier 220 may dynamically increase or decrease during flight to reflect flight phases where the biometric indicator is more or less predictive of the actual physiological state. For example, for the cruise flight phase where the pilot activity is expected to decrease, the weighting factor(s) assigned to the activity-based and/or vision-based indicators may be reduced while in the cruise flight phase while the weighting factor assigned to the biometric indicator may be increased when the biometric indicator is more probative of the actual physiological state relative to the activity-based and/or vision-based indicators. As described above, machine learning or other artificial intelligence techniques may be utilized to analyze historical relationships between the different physiological state indicators output by the pilot monitoring services 212, 214, 220 and an observed or manually-classified actual (or ground truth) contemporaneous physiological state with respect to the aircraft operational context and assign corresponding weighting factors to the different physiological state indicators that function as input variables to the equation or formula utilized to calculate an aggregate indicator of the physiological state as a function of the input physiological state indicators from the different pilot monitoring services 212, 214, 220 and the weighting factors.

Additionally, in a similar manner as described above, in some embodiments, the weighting factors assigned to the different modes of monitoring may be further influenced or adjusted historical correlations or relationships between the performance metrics associated with the different physiological state indicators output by the pilot monitoring services 212, 214, 220 and the observed contemporaneous physiological state with respect to those performance metrics. Depending on the embodiment, the performance metrics could be independent of or influenced by the current operational context, or the manner in which the performance metrics are utilized to influence the weighting factors could be influenced by the current operational context. For example, during early or initial stages of a flight where there is less baseline physiological measurement or visual data associated with the pilot upon which a statistically meaningful assessment of the physiological state can be made based upon that underlying data, the weighting factors associated with the biometric physiological state indicator and/or the vision-based physiological state indicator may be initially reduced, but progressively or dynamically increased over time as the performance metrics associated with the underlying data indicates a greater reliability or correlation to the actual physiological state, even though the flight phase or other contextual operational information associated with the aircraft is maintained substantially the same. Thereafter, when the flight phase or other operational context associated with the aircraft changes, the weighting factors associated with the different physiological state indicators may dynamically vary to reflect the current operational context in response to the change, while also dynamically varying over time within the same flight phase or operational context in accordance with the underlying performance metrics.

In one or more embodiments, the physiological state indicia output by the different pilot monitoring services 212, 214, 220 are realized as numerical values that are normalized to a common scale, which are then averaged or otherwise combined by the multimodal fusion service 230 using the different dynamic weighting factors associated therewith to arrive at an aggregate value for the physiological state. Different value ranges or thresholds may then be utilized to classify, categorize, or otherwise map the aggregate numerical value to a discrete physiological state from among the plurality of different potential physiological states. For example, the physiological state indicia output by the different pilot monitoring services 212, 214, 220 may be normalized to a scale with values ranging between 0 and 1, where a value of 1 represents a maximal level of alertness or awareness by the pilot and 0 represents a minimal level of alertness or awareness. Within the range of potential values defined by the scale, different subsets or subranges of values may be associated with or otherwise assigned to different discrete physiological states (e.g., values between 0.9 and 1 represent an alert state). In this regard, the subject matter described herein is not limited to any particular gradation or number of physiological states that may be defined within a range of potential values. For example, values between 0 and 0.1 may be designated or otherwise assigned to the deepest phase of sleep or other incapacity, while values between 0.1 and 0.2 are designated or otherwise assigned to a lighter phase of sleep, values between 0.2 and 0.3 are designated or otherwise assigned to a progressively lighter phase of sleep or drowsiness, and so on.

Still referring to FIG. 4, in exemplary embodiments, the multimodal monitoring process 400 continually monitors the aggregate indication of the current physiological state to detect or otherwise identify a change in the physiological state and automatically initiate one or more remedial actions in response to the change, where the actions are influenced by the classification or label assigned to the current physiological state (tasks 414, 416). For example, when the physiological state changes from a normal or alert state to an initial level of drowsiness (e.g., when values for the physiological state between 0.9 and 1 are assigned to the alert state and the aggregate value falls below 0.9), the processing system 202 and/or the multimodal fusion service 230 may automatically detect the change in physiological state and initiate one or more remedial actions that are configured to influence, counteract or otherwise respond to the drowsy physiological state. For example, the processing system 202 and/or the multimodal fusion service 230 may transmit or otherwise provide commands, signals or other instructions to one or more onboard systems 210 to generate one or more graphical and/or auditory alerts that are designed to prompt a response from the pilot or otherwise alter the pilot's physiological state to restore the pilot to a normal alert state. In addition to varying depending on the particular physiological state, the remedial actions may also vary depending on the current operational context or other factors. For example, in a cruise flight phase where autopilot is engaged, the processing system 202 and/or the multimodal fusion service 230 may disable, deemphasize or otherwise reduce the type and/or number of notifications or other remedial actions initiated because the change in the pilot's physiological state is less operationally significant. However, in a descent or approach flight phase, or when the pilot is manually flying the aircraft, the processing system 202 and/or the multimodal fusion service 230 escalate or increase the emphasis, type and/or number of notifications or other remedial actions initiated due to the increased operational significance of the pilot's physiological state with respect to the current flight phase or operational context. In this regard, it will be appreciated that in situations where the physiological state progressively trends towards increasing levels of drowsiness (or decreasing levels of alertness), the processing system 202 and/or the multimodal fusion service 230 may automatically escalate the graphical and/or auditory alerts (e.g., by increasing the volume, brightness, frequency and/or the like), as well as interact with one or more onboard systems 210 to influence operation of the aircraft. For example, the processing system 202 and/or the multimodal fusion service 230 may transmit or otherwise provide commands, signals or other instructions to the FMS 36, autopilot 42, autothrottle 44 and/or other onboard systems 210 to initiate autoland functionality or otherwise autonomously operate the aircraft as the aggregate indication of the pilot's current physiological state progresses away from an alert state or is otherwise maintained in a non-alert state for a prolonged period of time.

By virtue of aggregating or otherwise combining physiological state indicia using different modes of monitoring, the multimodal monitoring processes and systems described herein can more quickly and proactively respond to changes in the physiological state of a pilot or other vehicle operator while also avoiding false positives. For example, during phases of flight or periods of operation where less baseline activity by the pilot is expected, the physiological measurement data functions as a leading indicator that allows for the biometric physiological state indicia to identify a change to the physiological state of the pilot and proactively respond earlier in a manner that is more likely to prevent adverse progression of the pilot's physiological state. At the same time, in scenarios where activity-based or vision-based monitoring of the pilot may indicate a change in the pilot's physiological state, the physiological measurement data and corresponding biometric physiological state indicia may be utilized to confirm or otherwise verify that the pilot's physiological state is unchanged (e.g., the pilot is still alert and awake while appearing inactive) and suppress or otherwise fail to initiate remedial actions that would otherwise be unnecessary by virtue of the biometric monitoring output being weighted more heavily than the activity-based or vision-based monitoring outputs at that particular time or operational context. Thus, by varying the relative weighting or manner in which the physiological state indicia from different modes of monitoring are combined, and varying the weighting or manner with respect to the current operational context, time and/or other factors that influence the reliability or predictivity of the respective monitoring mode, the resulting aggregate physiological state indicator improves the ability to monitor the pilot or other vehicle operator in a manner that allows the system to respond to changes in the physiological state more quickly with greater confidence.

For the sake of brevity, conventional techniques related to graphical user interfaces, autopilot, autothrottle, flight control systems, flight management systems, avionics systems, and other functional aspects of the systems (and the individual operating components of the systems) may not be described in detail herein. Furthermore, the connecting lines shown in the various figures contained herein are intended to represent exemplary functional relationships and/or physical couplings between the various elements. It should be noted that many alternative or additional functional relationships or physical connections may be present in an embodiment of the subject matter.

The subject matter may be described herein in terms of functional and/or logical block components, and with reference to symbolic representations of operations, processing tasks, and functions that may be performed by various computing components or devices. It should be appreciated that the various block components shown in the figures may be realized by any number of hardware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices. Furthermore, embodiments of the subject matter described herein can be stored on, encoded on, or otherwise embodied by any suitable non-transitory computer-readable medium as computer-executable instructions or data stored thereon that, when executed (e.g., by a processing system), facilitate the processes described above.

The foregoing description refers to elements or nodes or features being "coupled" together. As used herein, unless expressly stated otherwise, "coupled" means that one element/node/feature is directly or indirectly joined to (or directly or indirectly communicates with) another element/node/feature, and not necessarily mechanically. Thus, although the drawings may depict one exemplary arrangement of elements directly connected to one another, additional intervening elements, devices, features, or components may be present in an embodiment of the depicted subject matter. In addition, certain terminology may also be used herein for the purpose of reference only, and thus are not intended to be limiting.

The foregoing detailed description is merely exemplary in nature and is not intended to limit the subject matter of the application and uses thereof. Furthermore, there is no intention to be bound by any theory presented in the preceding background, brief summary, or the detailed description.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the subject matter in any way. Rather, the foregoing detailed description will provide those skilled in the art with a convenient road map for implementing an exemplary embodiment of the subject matter. It should be understood that various changes may be made in the function and arrangement of elements described in an exemplary embodiment without departing from the scope of the subject matter as set forth in the appended claims. Accordingly, details of the exemplary embodiments or other limitations described above should not be read into the claims absent a clear intention to the contrary.

## Claims

1. A method of assisting operation of a vehicle, the method comprising:
determining a first value corresponding to a first physiological state of a plurality of different physiological states for a vehicle operator based on measurement data from one or more sensing devices configured to measure one or more characteristics associated with the vehicle operator;
determining a second value corresponding to a second physiological state of the plurality of different physiological states for the vehicle operator based on output signals from one or more user interface elements;
determining an aggregate indicator of a current physiological state of the plurality of different physiological states for the vehicle operator as a function of the first value and the second value using a first weighting factor associated with the first value and a second weighting factor associated with the second value, wherein the first weighting factor and the second weighting factor are different and at least one of the first weighting factor and the second weighting factor varies during operation of the vehicle; and
automatically initiating a remedial action based on the aggregate indicator.

2. The method of claim 1, further comprising determining a third value corresponding to a third physiological state of the plurality of different physiological states for the vehicle operator based on one or more captured images, wherein determining the aggregate indicator comprises determining the aggregate indicator as a function of the first value, the second value and the third value using the first weighting factor associated with the first value, the second weighting factor associated with the second value and a third weighting factor associated with the third value.

3. The method of claim 1, further comprising:
obtaining status information indicative of a current operational context associated with the vehicle from one or more onboard systems; and
determining the at least one of the first weighting factor and the second weighting factor based on the current operational context.

4. The method of claim 3, wherein:
the vehicle comprises an aircraft;
the current operational context comprises a flight phase of the aircraft; and
the at least one of the first weighting factor and the second weighting factor varies in response to a change in the flight phase of the aircraft.

5. The method of claim 1, further comprising:
determining one or more performance metrics associated with the measurement data from the one or more sensing devices; and
determining the at least one of the first weighting factor and the second weighting factor based on the one or more performance metrics.

6. The method of claim 1, wherein determining the first value comprises:
obtaining a first set of one or more physiological measurement data samples from a first sensing device configured to measure a first physiological characteristic of the vehicle operator; and
determining the first value based at least in part on the first set of one or more physiological measurement data samples.

7. The method of claim 6, further comprising obtaining a second set of one or more physiological measurement data samples from a second sensing device configured to measure a second physiological characteristic of the vehicle operator, wherein:
the second physiological characteristic is different from the first physiological characteristic; and
determining the first value comprises determining the first value based at least in part on the first set of one or more physiological measurement data samples from the first sensing device and the second set of one or more physiological measurement data samples from the second sensing device.

8. The method of claim 7, wherein:
determining the first value comprises:
determining a third value for the current physiological state of the vehicle operator based at least in part on the first set of one or more physiological measurement data samples from the first sensing device;
determining a fourth value for the current physiological state of the vehicle operator based at least in part on the second set of one or more physiological measurement data samples from the second sensing device; and
determining an aggregate biometric indicator for the first physiological state based at least in part on the third value and the fourth value; and
determining the aggregate indicator of the current physiological state comprises determining the aggregate indicator of the current physiological state as a function of the aggregate biometric indicator and the second value using the first weighting factor associated with the aggregate biometric indicator and the second weighting factor associated with the second value.

9. The method of claim 1, further comprising progressively increasing the first weighting factor over time during operation of the vehicle.

10. The method of any preceding claim, wherein the vehicle comprises an aircraft and the at least one of the first weighting factor and the second weighting factor dynamically varies with respect to a current flight phase of the aircraft.

11. The method of claim 1, wherein determining the second value comprises identifying the second physiological state of the plurality of different physiological states in a manner that is influenced by the output signals from the one or more user interface elements over a monitoring period of time, wherein the vehicle comprises an aircraft and the monitoring period of time varies with respect to a current flight phase of the aircraft.

12. The method of claim 1, wherein the vehicle comprises an aircraft and automatically initiating the remedial action comprises automatically initiating autonomous operation of the aircraft based on the aggregate indicator.

13. The method of any preceding claim, wherein automatically initiating the remedial action comprises automatically generating an alert intended to influence the current physiological state of the vehicle operator.

14. A computer-readable medium having computer-executable instructions stored thereon that, when executed by a processing system, cause the processing system to:
determine a first value corresponding to a first physiological state of a plurality of different physiological states for a vehicle operator based on measurement data from one or more sensing devices configured to measure one or more characteristics associated with the vehicle operator;
determine a second value corresponding to a second physiological state of the plurality of different physiological states for the vehicle operator based on output signals from one or more user interface elements;
determine an aggregate indicator of a current physiological state of the plurality of different physiological states for the vehicle operator as a function of the first value and the second value using a first weighting factor associated with the first value and a second weighting factor associated with the second value, wherein the first weighting factor and the second weighting factor are different and at least one of the first weighting factor and the second weighting factor varies during operation of a vehicle; and
automatically initiate a remedial action based on the aggregate indicator.

15. An aircraft system comprising:
a sensing device to obtain physiological measurement data for a pilot of an aircraft;
a user interface element onboard the aircraft to provide signals responsive to the pilot interacting with the user interface element; and
a processing system coupled to the sensing device and the user interface element to provide a multimodal monitoring service to:
determine a biometric indication of a first physiological state of a plurality of different physiological states for the pilot based at least in part on the physiological measurement data;
determine an activity-based indication of a second physiological state of the plurality of different physiological states for the pilot based on the signals from the user interface element;
determine an aggregate indicator of a current physiological state of the plurality of different physiological states for the pilot as a function of the biometric indication and the activity-based indication using a first weighting factor associated with the biometric indication and a second weighting factor associated with the activity-based indication, wherein the first weighting factor and the second weighting factor are different and at least one of the first weighting factor and the second weighting factor varies during operation of the aircraft; and
automatically initiate a remedial action based on the aggregate indicator.
